# EUROPÄISCHE PATENTSCHRIFT

(11) **EP 3 403 618 B1**
(45) Veröffentlichungstag und Bekanntmachung des Hinweises auf die Patenterteilung: **08.01.2020**
(21) Anmeldenummer: 18172407.1
(22) Anmeldetag: 15.05.2018
(51) Int. Cl.: A61F 2/36

(54) **KUPPLUNGSVORRICHTUNG ZUM VERBINDEN VON PROTHESENKOMPONENTEN ÜBER EINEN SELBSTHEMMENDEN KLEMMSITZ**
COUPLING DEVICE FOR THE CONNECTION OF PROSTHETIC COMPONENTS VIA A SELF-LOCKING PRESS FIT
DISPOSITIF D'ACCOUPLEMENT PERMETTANT DE RACCORDER DES COMPOSANTS DE PROTHÈSE À UN SIÈGE DE SERRAGE AUTOBLOQUANT

(30) Priorität: 16.05.2017 DE 102017004911
(43) Veröffentlichungstag der Anmeldung: 21.11.2018
(73) Patentinhaber: Aristotech Holding GmbH, 10719 Berlin (DE)
(72) Erfinder: Anapliotis, Emmanuel, 14195 Berlin (DE)
(74) Vertreter: Bittner, Thomas L.

(56) Entgegenhaltungen:
- EP-A1- 1 293 179
- EP-A1- 2 915 506
- EP-A2- 0 562 782
- WO-A1-2014/140639
- US-A1- 2006 188 845
- US-A1- 2011 087 335

## Beschreibung

Die Erfindung betrifft eine Kupplungsvorrichtung, mit welcher Komponenten einer Prothese mittels einer Konusverbindung unter Ausbildung eines selbsthemmenden Klemmsitzes gegeneinander verklemmt und dadurch miteinander verbunden werden können. Im Speziellen betrifft die Erfindung eine Kupplungsvorrichtung für die Konusverbindung zwischen dem Hüftschaft und dem Steckkopf einer Hüftgelenksendoprothese.

### Hintergrund

Insbesondere in der Hüftendoprothetik hat sich die Konussteckverbindung zwischen Hüftkopf und Hüftschaft als stabile selbsthemmende Klemmverbindung bewährt. Darüber hinaus finden Konussteckverbindungen aufgrund ihrer Klemmeigenschaften auch in anderen prothetischen Versorgungssystemen Verwendung.

Probleme mit der Erneuerung einer Konussteckverbindung treten allerdings dann auf, wenn eine der Prothesenkomponente des Prothesensystems ersetzt werden muss.

Beispielsweise geht in der Regel eine Pfannenrevision mit dem Austausch des Gelenkkopfes einher. Um in einem solchen Fall nicht auch noch den liegenden Schaft explantieren und gegen einen mit dem Konus des neuen Gelenkkopfes korrespondierenden Schaft austauschen zu müssen, sind Adapterhülsen entwickelt worden, mit denen der neue Gelenkkopf mit dem in situ verbliebenen Schaft über eine Konus-Steckverbindung sicher verbunden werden kann.

Aus dem Stand der Technik ist eine Vielzahl von konusförmigen Adapterhülsen bekannt, die zu unterschiedlichen Zwecken konzipiert wurden.

Die DE 40 08 563 A1 offenbart eine solche Konussteckverbindung, bei der ein konusförmiger Adapter als Kopplungsinstrument zwischen Gelenkkopf und Schafthals verwendet wird, um Gelenkköpfe und Schafthälse mittels Reibschluss verbinden zu können, wobei der Gelenckopf und der Schafthals aus unterschiedlichen Materialien hergestellt sein können und auch unterschiedliche Konizitäten aufweisen können.

Vormontierte Adapter für Konussteckverbindungen sind aus den Schriften CH 676 922 A5 und WO 2005/120596 A1 bekannt. Letztere offenbart zudem die Herstellung eines solchen konisch geformten Adapters aus biokompatiblen Kunststoffen.

In WO 2014/140639 A1 ist ein Probeimplantatsystem beschrieben, in welchem verschiedene Köpfe auf einen Prothesenschaft probeweise aufgesetzt werden können. Zum Schutz des konusförmigen Halses des Schafts ist eine abnehmbare Schutzhülse vorgesehen, welche eine starre Außenseite und eine weichere, verformbare Innenseite aufweist. Dazu ist die Hülse aus einem starren äußeren Teil und einem mit diesem verbundenen, verformbaren inneren Teil gebildet.

Es ist auch bekannt, dass die Oberfläche eines Schaftkonus nach der Entfernung des Gelenkkopfes nicht mehr die Güte besitzt, die ein spannungsspitzenfreies Aufstecken eines Ersatzkopfes erlaubt, In Folge dieser Spannungsspitzen können im Gelenkkopf Risse und Brüche entstehen, was wiederum zu einer erneuten Revision des Ersatzgelenkkopfes führen kann.

Dieser Problematik nimmt sich die DE 103 03 660 B4 an, die für die Versorgung von liegenden Schäften mit neuen Gelenkköpfen einen Adapter angibt, der dafür Sorge trägt, dass die Spannungen gleichmäßig über die Kontaktfläche verteilt oder zumindest in den oberen Bereich des Konus abgeleitet werden. Hierzu trägt der Adapter von seiner unteren breiteren Seite eine nach oben verlaufende Nut und sein Innenkonus ist im Verlauf gewölbt ausgebildet.

Die DE 199 04 437 A1 beschreibt einen konischen Adapter, der besondere Elastizitäts- und Dämpfungseigenschaften aufweist. Zur gleichmäßigeren Ableitung der Spannung zwischen dem Konus des Schaftes und dem Kugelkopf ist der Adapter als poröser Wickelkörper ausgebildet. Mit der offenbarten Konstruktion ist es möglich, beschädigte Kugelköpfe auszuwechseln, ohne dass der Schaft entfernt werden muss, selbst wenn der Zustand der Oberfläche des Konus ein direktes Aufsetzen der Kugelköpfe nicht mehr zulassen würde.

Die US 2008/0262626 A1 offenbart eine Lösung zur Versorgung des Oberschenkelhalskopfes mit einer Hüftkopfprothese, bei der auf das präparierte Schenkelhalsende eine geschlitzte Hülse aufgesteckt wird, auf die dann ein künstlicher Hüftkopf aufgesteckt wird.

Zum Ausgleich von Winkelfehlern des Konus am Prothesenhals bei gleichzeitiger Erhöhung der Prothesenlebensdauer stellt die EP 2 459 124 B4 einen Adapter zur Verfügung, der zwar grundlegend einen konischen Formkörper, jedoch federnde und tragende Abschnitte aufweist, aufgrund derer die Kraft in einen definierten Bereich im Gelenkkopf umgeleitet wird und die lasttragenden Bereiche dadurch entlastet werden.

In EP 2 915 506 A1 ist eine Hülse zur Verbindung eines Schafts einer Hüftgelenksendoprothese mit einem entsprechenden Kopf beschrieben, welche zwei ineinander angeordnete konische Hülsen umfasst. Die innere Hülse ist länger als die äußere Hülse, so dass die äußere Hülse nicht in Kontakt mit dem Randbereich des Kopfes kommt, um Belastungen weiter in das Innere des Kopfes zu leiten.

Ein Problem bei konustragenden Prothesen ist, dass die Konusgeometrien nicht standardisiert sind, so dass die Maße der Konen unterschiedlicher Hersteller deutlich voneinander abweichen können. Auch Angaben wie "12/14 Eurokonus" oder "12/14 Standardkonus" bezeichnen keine einer Norm unterworfene Konusgeometrie, die vom Hersteller unabhängige und standardisierte Maße garantiert.

Wenn dann auch noch der Konus der Prothese beschädigt ist oder dessen Toleranzen außerhalb der produktüblichen Standardabweichung liegen, kann eine sichere und langzeitstabile Konussteckverbindung zwischen den Prothesenkomponenten auch bei Verwendung eines Konus-Adapters nicht mehr gewährleistet werden.

### Zusammenfassung

Aufgabe der Erfindung ist es daher, ein Adaptersystem für einen Konussteckverbindung unter Prothesenkomponenten anzugeben, das insbesondere bei defekten Konen oder Konen, deren Geometrien nicht bekannt oder ermittelbar sind, zu einem langzeitstabilen und sicheren Klemmsitz zwischen den Prothesenkomponenten führt.

Zur Lösung der Aufgabe wird eine Kupplungsvorrichtung zur Verbindung von Prothesenkomponenten angegeben, mit welcher ein selbsthemmender Klemmsitz auf einem als Konus ausgebildeten Ende einer in ein Knochengewebe eingesetzten ersten Prothesenkomponente erzeugbar ist und die einen äußeren Kupplungskörper mit mindestens einer Fassung mit einer konisch zulaufenden Innenwandung und eine Spreizhülse mit einer einen Außenkonus bildenden Mantelfläche und einer einen Innenkonus bildenden Wirkfläche umfasst.

Hierbei ist die Spreizhülse in der Fassung im Kupplungskörper um deren Längsachse rotierbar gelagert und zur Aufnahme des Konus der ersten Prothesenkomponente hergerichtet.

Das Kupplungssystem zeichnet sich dadurch aus, dass die Spreizhülse zur Ausbildung des selbsthemmenden Klemmsitzes radial nach außen dehnbar ausgestaltet ist, so dass beim Einfügen des Konus der ersten Prothesenkomponente in die Spreizhülse eine radiale Aufweitung der Spreizhülse erfolgt, wodurch der selbsthemmende Klemmsitz zwischen dem Konus der ersten Prothesenkomponente und der Kupplungsvorrichtung herstellbar ist.

Bei der Ausbildung des selbsthemmenden Klemmsitzes wirken bei der erfindungsgemäßen Kupplungsvorrichtung mehrere Flächenpaarungen miteinander.

Folgende Flächenpaarungen sind dabei mindestens an der Ausbildung des Klemmsitzes zwischen erster Prothesenkomponente und der Kupplungsvorrichtung beteiligt:
a) die Konusoberfläche der ersten Prothesenkomponente und die Wirkfläche des Innenkonus der Spreizhülse sowie
b) die Mantelfläche der Spreizhülse und die Innenwandung der Fassung.
   Bei der Kopplung mit einer zweiten Prothesenkomponente ist zusätzlich noch mindestens nachstehende Flächenpaarung an einer Klemmverbindung beteiligt:
c) die Außenfläche der konusförmigen Außenkontur des Kupplungskörpers mit der Innenfläche einer konusförmigen Innenkontur in einer zweiten Prothesenkomponente.

Letztere Ausgestaltung betrifft insbesondere einen konusförmigen Adapter, welcher zwischen einem Gelenkkopf und einem Schafthals einer Hüftendoprothese eingesetzt wird, um den Gelenkkopf auf dem Schaftkonus festzusetzen.

Weitere Einsatzgebiete einer erfindungsgemäßen Kupplungsvorrichtung betreffen Verbindungselemente, die dazu hergerichtet sind, Prothesenkomponenten wie Knochennägel miteinander oder mit Schäfte von Gelenkprothesen zu verbinden.

Die konstruktive Dimensionierung von Spreizhülse zu Fassung ist bei der erfindungsgemäßen Konstruktion derart ausgelegt, dass die Spreizhülse im nicht mit dem Konus der ersten Prothesenkomponente verklemmten Zustand (freier Zustand) in der Fassung frei rotierbar ist. Hierzu ist der Außenkonus der Spreizhülse vorteilhafterweise geringfügig kleiner dimensioniert als der Mutterkonus der Fassung, so dass zwischen der Spreizhülse und der Fassung Spiel besteht.

Erst mit dem Einsetzen des Konus der ersten Prothesenkomponente in den Innenkonus der Spreizhülse finden ein Aufweiten der Spreizhülse statt, wodurch die Hülse aufgedehnt und deren Mantelfläche gegen die Innenwandung der Fassung gepresst und dadurch ein selbsthemmender Klemmsitz ausgebildet wird.

Eine erste Prothesenkomponente ist insbesondere ein Hüftschaft oder ein intramedullär gesetzter Knochennagel, der einen oder mehrere konusförmige Anschlussbereiche aufweist.

Die erfindungsgemäße Kupplungsvorrichtung hat gegenüber dem Stand der Technik den Vorteil, dass Defekte, Verformungen oder Toleranzabweichungen bei Konen mittels der Kupplungsvorrichtung überbrückt und dadurch mit Ersatzkomponenten verbunden werden können, so dass die noch stabil im Knochen liegende Prothesenkomponente - bei entsprechender Einschätzung des Arztes - in situ belassen werden kann. Dadurch können zum Wohle des Patienten Totalrevisionen vermieden und die Belastung durch einen erhöhten operativen Aufwand gesenkt werden, was wiederum die Heilungszeiten verkürzt und die Heilungsaussichten verbessert.

Auch hat sich gezeigt, dass unter Verwendung einer erfindungsgemäßen Kupplungsvorrichtung ein geringerer Kraftaufwand beim Zusammenfügen der Prothesenkomponenten erforderlich ist, um eine langzeitstabile selbsthemmende Klemmwirkung zwischen den Prothesenkomponenten zu erzielen.

Um eine für die Aufweitbarkeit der Spreizhülse erforderliche radiale Dehnbarkeit zu erhalten, weist die Spreizhülse vorzugsweise mindestens einen von einem Rand ausgehenden und in Längsrichtung der Spreizhülse verlaufenden Schlitz auf. In einer besonderen Ausgestaltung ist die Spreizhülse in Längsrichtung durchgehend geschlitzt, so dass die Spreizhülse als Spaltring ausgebildet ist.

Um eine gleichmäßigere Kraftverteilung während der Klemmsitzausbildung zu erzielen, sind in einer weiteren Ausgestaltungsform in der Spreizhülse umlaufend in Längsrichtung verlaufende Schlitze eingerichtet, die vorteilhafterweise alternierend vom Rand des breiteren Endes und vom Rand des schmaleren Endes ausgehen, so dass die Spreizhülse eine mäanderförmige Struktur erhält.

Um die Klemmsitzfestigkeit zu erhöhen, kann die Konizität des Innenkonus der Spreizhülse ein geringfügig kleineres Maß als die Konizität des Konus der ersten Prothesenkomponente aufweisen, wodurch die Spreizhülse beim Einfügen des Konus zwangsweise geweitet wird.

In einer weiteren Ausgestaltungsform ist die Spreizhülse in der Fassung des Kupplungskörpers vormontiert und gegen Herausgleiten aus der Fassung in axialer Richtung gesichert.

In einer ersten Alternative ist zur Halterung der Spreizhülse in der Fassung auf der Mantelfläche der Spreizhülle eine radial umlaufende Wulst eingerichtet, die mit einer radial umlaufenden Nut in der Innenwandung der Fassung in Eingriff bringbar ist. Der Nut-Wulst-Eingriff ist derart ausgelegt, dass beim Einsetzen der Spreizhülse die Wulst in die Nut einschnappt, ohne jedoch die Spreizhülse mit der Fassung zu verklemmen, d.h. die Rotierbarkeit der Spreizhülse um deren Längsachse bleibt mit dem Nut-Wulst-Eingriff erhalten.

In einer zweiten Alternative wird die Halterung der Spreizhülse in der Fassung dadurch erreicht, dass in der Aufnahmeöffnung der Fassung eine radial umlaufende Hinterschneidung eingerichtet ist, auf die sich fassungsinnenseitig die in die Fassung eingesetzte Spreizhülse mit deren unterem Rand aufstützt. Wesentlich ist, dass die Hinterschneidungsöffnung größer als die in diesem Bereich befindliche Öffnung des Spreizhülsen-Innenkonus ist und dass die Spreizhülse nur in deren äußeren Randbereich von der Hinterschneidung umgriffen wird. Der innere Randbereich der Spreizhülse steht somit über den Rand der Hinterschneidung über, so dass die Hinterschneidung das Einfügen des Konus der ersten Prothesenkomponente in den Innenkonus der Spreizhülse nicht behindert.

In dem der Aufnahmeöffnung gegenüberliegenden Kopfbereich der Fassung ist vorteilhafterweise auf der Innenwandung eine stufenartige und radial umlaufende Einschnürung eingerichtet, durch die die eingesetzte Spreizhülse in Abstand zum Kopfbereich der Fassung gehalten wird. Vorteilhafterweise ist im Kopfbereich der Fassung eine zur Umgebung offene Bohrung eingerichtet, durch die einerseits Luft entweichen kann, wenn der Konus der ersten Prothesenkomponente eingeführt wird. Andererseits kann über die Bohrung ein entsprechendes Instrument eingesetzt werden, mit welchem der Kupplungskörper von einer der Prothesenkomponenten abgedrückt werden kann.

In einer ersten Anwendungsform weist der Kupplungskörper eine konusförmige Außenkontur auf, die mit einer konusförmigen Innenkontur in einer zweiten Prothesenkomponente in einen selbsthemmenden Klemmsitz bringbar ist. Diese Anwendungsform entspricht in ihrer Außenform und Zweckbestimmung im Wesentlichen den bekannten konusförmigen Adaptern, die verwendet werden, um zwischen einem Gelenkkopf und einem Schaftkonus einen selbsthemmenden Klemmsitz herzustellen. Der Gelenkkopf stellt in diesem Ausführungsbeispiel dann die zweite Prothesenkomponente dar.

In einer zweiten Anwendungsform weist die Kupplungsvorrichtung einen zylinderförmigen Kupplungskörper auf, an dessen Enden jeweils eine Fassung mit einer darin gelagerten Spreizhülse eingerichtet ist. Eine solche Kupplungseinrichtung ist geeignet, zwei Prothesenkomponenten über deren konisch geformte Endbereiche miteinander selbsthemmend zu verklemmen. Beispielhaft findet eine solche Kupplungsvorrichtung Anwendung bei der intramedullären Verlängerung von liegenden Hüftschäften mit Knochennägeln in dorsaler Richtung.

Der zylinderförmige Kupplungskörper kann geradlinig als auch in gebogener oder gewinkelter Form ausgebildet sein. Letztere Konstruktionen sind geeignet, bei geforderter dauerhafter oder zeitlich begrenzter Arthrodese den Gelenkbereich in angewinkelter Stellung zu überbrücken.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: einen beispielhaften Aufbau eines Verbunds von Prothesenkomponenten, bei dem eine erste und eine zweite Prothesenkomponente über eine erfindungsgemäße Kupplungsvorrichtung miteinander verbunden sind,
- Fig. 2a-c: eine erste Ausführungsvariante eines äußeren Kupplungskörpers,
- Fig. 3a-b: eine erste Ausführungsvariante einer Spreizhülse,
- Fig. 4: eine erste Ausführungsvariante einer Kupplungsvorrichtung zusammengesetzt aus Spreizhülse und Kupplungskörper,
- Fig. 5a-b: eine zweite Ausführungsvariante eines äußeren Kupplungskörpers,
- Fig. 6a-c: eine zweite Ausführungsvariante einer Spreizhülse,
- Fig. 7: eine zweite Ausführungsvariante einer Kupplungsvorrichtung zusammengesetzt aus Spreizhülse und Kupplungskörper und
- Fig. 8: eine alternative Ausführungsform einer Kupplungsvorrichtung mit zwei Spreizhülleneinsätzen, eingerichtet an den gegenüberliegenden Enden der Kupplungsvorrichtung.

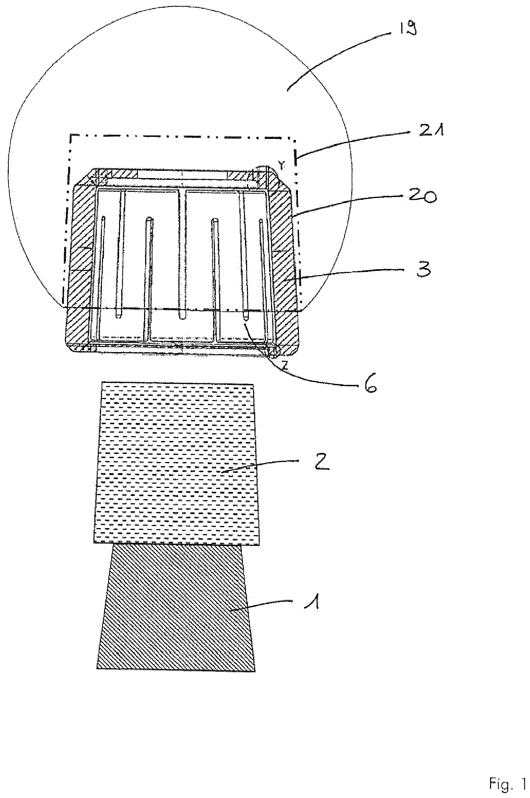

Die Fig. 1 zeigt einen beispielhaften Aufbau einer Prothese, bei der eine erste Prothesenkomponente 1 mit einem konusförmigen Anschlußbereich 2 mit einer zweiten Prothesenkomponente 19 mit einer konusförmigen Innenkontur 21 über zwei selbsthemmende Klemmsitze miteinander gekoppelt werden. Der Verbund der Prothesenkomponenten 1 und 2 wird durch eine erfindungsgemäße Kupplungsvorrichtung hergestellt, die aus einem äußeren Kupplungskörper 3 mit einer konusförmigen Außenkontur 20 und einer in einer Fassung 4 eingerichteten Spreizhülse 6, die geschlitzt ist, zusammengesetzt ist.

Die konusförmige Spreizhülse 6 ist in der konusförmigen Fassung 4 des Kupplungskörpers 3 rotierbar gelagert und durch die Hinterschneidung 17 gegen ein Herausgleiten aus der Fassung 4 gesichert.

Zur Erzeugung des Klemmsitzes zwischen der ersten Prothesenkomponente 1 und der Kupplungsvorrichtung wird der Konus 2 der ersten Prothesenkomponenten 1 in den Innenkonus 9 der Spreizhülse 6 eingeschoben, wodurch die Spreizhülse 6 aufgeweitet und deren Mantelfläche 8 gegen die Innenwandung 5 der Fassung 4 gepresst wird, was dazu führt, dass der Konus 2 der ersten Prothesenkomponente 1 und der Kupplungskörper 3 miteinander verklemmt werden.

Der zweite Klemmsitz wird beim Aufsetzen der zweiten Prothesenkomponente 19 auf die konusförmige Außenkontur 20 des Kupplungskörpers 3 ausgebildet, indem der konusförmige Kupplungskörper 3 in eine konusförmige Aufnahme 21 der zweiten Prothesenkomponente 19 eingeschlagen wird.

In der Fig. 1 sind als erste Prothesenkomponente 1 beispielhaft ein Konus 2 eines Hüftschaftes und als zweite Prothesenkomponente 19 der Prothese die auf den Konus 21 des Hüftschaftes aufzuschlagende Gelenkkugel dargestellt.

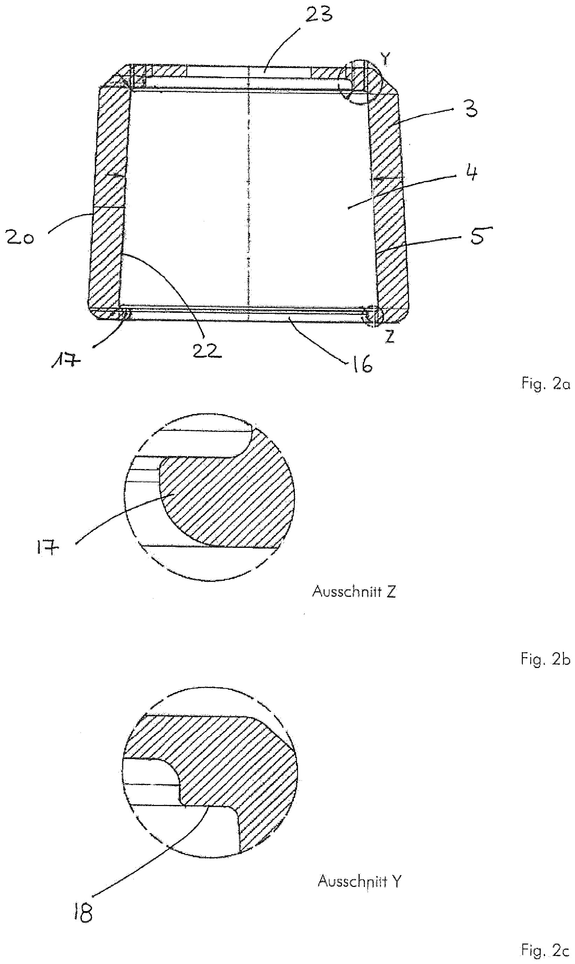

Die Fig. 2a - c zeigen eine erste Ausgestaltungsform eines äußeren Kupplungskörpers 3. Der dargestellte Kupplungskörper 3 ist in seiner äußeren Form 20 konusförmig und entspricht damit in seiner Außenkontur den in der Hüftendoprothetik bekannten Adaptern, die zur Verbindung von Kugelköpfen und Hüftschäften eingesetzt werden. Die im Innenraum des Adapters eingerichtete Fassung 4 ist ebenfalls konusförmig ausgebildet. Im abgebildeten Fall sind der Konuswinkel der äußeren Konuskontur 20 und der des Mutterkonus 22 identisch. Die Konuswinkel können aber auch voneinander abweichende Größen annehmen.

Im Bereich des breiten Endes des Kupplungskörpers 3 befindet sich die Aufnahmeöffnung 16 der Fassung 4, in der eine umlaufende Hinterschneidung 17 eingerichtet ist, durch die die Aufnahmeöffnung 16 gegenüber dem daran anschließenden Bereich der Fassung 4 einen verengten Querschnitt aufweist. Die Hinterschneidung 17 ist im Detail nochmals in der Fig. 2b wiedergegeben.

Im Kopfbereich der Fassung 4 ist eine stufenartig ausgestaltete und radial umlaufende Einschnürung 18 eingerichtet (Fig. 2c), durch die die Spreizhülse (nicht dargestellt) im Abstand zum Kopfbereich der Fassung 4 gehalten wird. Die im Kopfbereich der Fassung 4 zusätzlich befindliche Bohrung 23 hat mehrere Funktionen. Einerseits ermöglicht sie, dass Luft entweichen kann, wenn der Konus der ersten Prothesenkomponente eingeführt wird, andererseits kann über die Bohrung 23 ein entsprechendes Instrument eingesetzt werden, mit welchem der Kupplungskörper 3 von einer der Prothesenkomponenten abgedrückt werden kann.

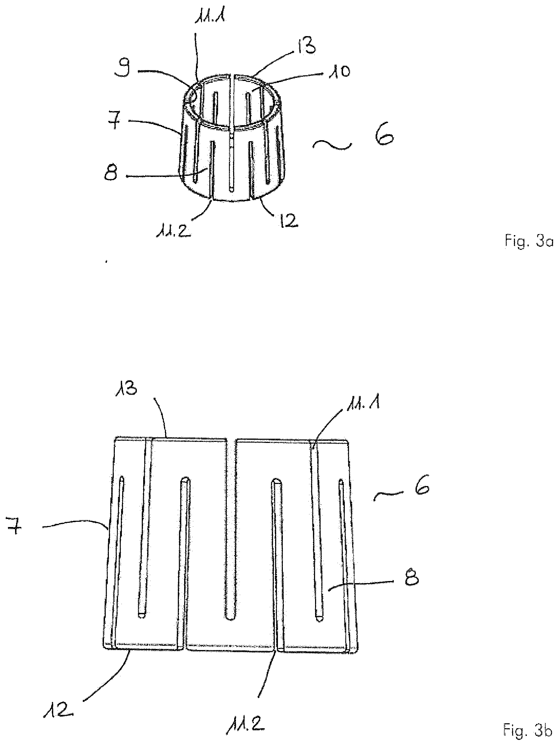

Die Fig. 3a und b zeigen eine Spreizhülse 6, die mit dem in den Fig. 2a-c dargestellten Kupplungskörper 6 kompatibel ist.

Die dargestellte Spreizhülse 6 hat einen Außenkonus 7 mit einer Mantelfläche 8 sowie einen Innenkonus 9 mit einer Wirkfläche 10. Zur Erzeugung einer radialen Dehnbarkeit ist die Spreizhülse 6 in Längsrichtung geschlitzt, wobei umlaufend in der Spreizhülse 6 in Längsrichtung verlaufende Schlitze 6 eingearbeitet sind, die alternierend vom Rand 12 des breiteren Endes und vom Rand 13 des schmaleren Endes ausgehen, so dass die Spreizhülse 6 eine mäanderförmige Struktur aufweist.

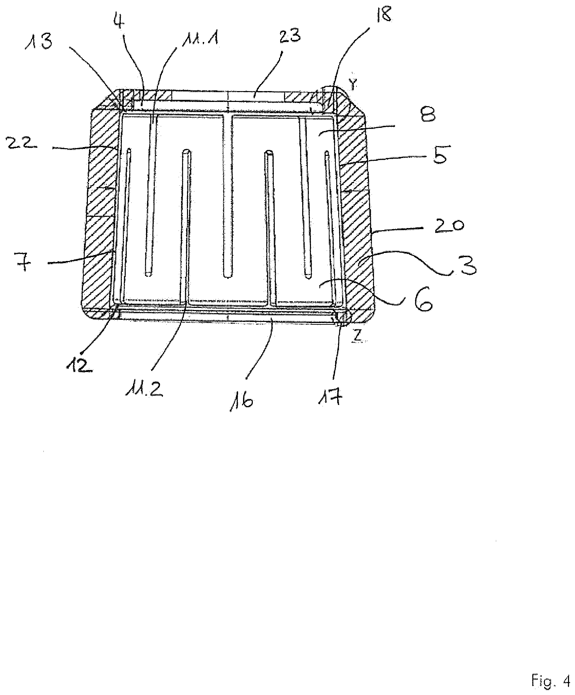

Die Fig. 4 zeigt eine Alternative einer Kupplungsvorrichtung, bei der eine Spreizhülse 6, wie sie in den Fig. 3a und b wiedergegeben ist, eingesetzt ist in die Fassung 4 eines Kupplungskörpers 3 in einer Ausgestaltungsform gemäß der Fig. 2a - c.

Die Spreizhülse 6 ist durch Einrichten eines Spiels zwischen der Mantelfläche 8 des Außenkonus 7 und der Innenwandung 5 des Kupplungskörpers 3 rotierbar in der Fassung 4 gelagert und gegen ein Herausgleiten aus der Fassung 4 durch die Hinterschneidung 17 gesichert, wobei sich der unteren Rand 12 der Spreizhülse 6 fassungsinnenseitig auf der Hinterschneidung 17 aufstützt.

Wesentlich bei dieser Ausgestaltungsform ist, dass die Hinterschneidungsöffnung größer als die in große Konus-Öffnung des Innenkonus 9 der Spreizhülse 6 ist, so dass die Spreizhülse 6 nur in deren äußeren Randbereich 12 von der Hinterschneidung 17 umgriffen wird. Der innere Randbereich 12 der Spreizhülse 6 steht somit in Richtung der Längsachse der Kupplungsvorrichtung über den Rand der Hinterschneidung 17 über, so dass die Hinterschneidung 17 das Einfügen eines Konus 2 einer ersten Prothesenkomponente 2 (nicht dargestellt) in den Innenkonus 9 der Spreizhülse 6 nicht behindert.

Kopfseitig der Fassung 4 verhindert die Einschnürung 18 ein Anschlagen des oberen Randes 13 der Spreizhülse 6 an den Endbereich des Kupplungskörpers 3.

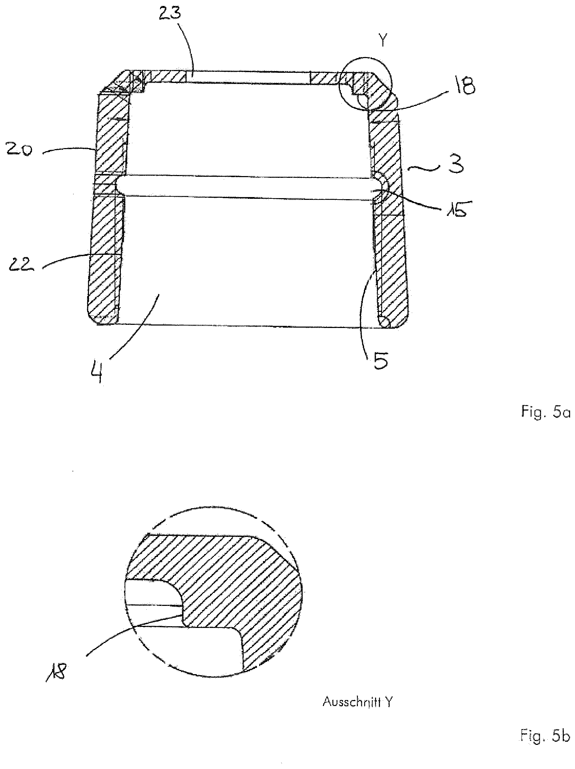

Die Fig. 5a und 5b zeigen eine zweite Ausgestaltungsform eines äußeren Kupplungskörpers 3. Der dargestellte Kupplungskörper 3 ist in seiner äußeren Form 20 ebenso konusförmig ausgebildet und entspricht auch in dieser Variante in seiner Außenkontur den in der Hüftendoprothetik bekannten Adaptern, die zur Verbindung von Kugelköpfen und Hüftschäften eingesetzt werden.

Auch die im Innenraum des adapterförmigen Kupplungskörpers 3 eingerichtete Fassung 4 ist konusförmig ausgebildet. Im dargestellten Fall sind wie bei der vorab beschriebenen Ausgestaltungsvariante der Konuswinkel der äußeren Konuskontur 20 und der des Mutterkonus 22 identisch. Die Konuswinkel können aber auch bei dieser Ausgestaltungsform voneinander abweichende Größen annehmen.

In der Innenwandung 5 der Fassung 4 ist eine radial umlaufende Nut 15 eingerichtet, mit der eine radial ausgerichtete Wulst 14 auf der Mantelfläche 8 einer eine Spreizhülse 6 in Eingriff bringbar ist.

Im Kopfbereich der Fassung 4 ist wiederum eine stufenartig ausgestaltete und radial umlaufende Einschnürung 18 eingerichtet (Fig. 5b), durch die die Spreizhülse (nicht dargestellt) im Abstand zum Kopfbereich der Fassung 4 gehalten wird. Die im Kopfbereich der Fassung 4 zusätzlich befindliche Bohrung 23 hat auch hier die Funktionen, dass Luft entweichen kann, wenn der Konus der ersten Prothesenkomponente eingeführt wird, oder dass über die Bohrung 23 ein entsprechendes Instrument eingesetzt werden kann, um den Kupplungskörper 3 von einer der Prothesenkomponenten 1 und/oder 19 abzudrücken.

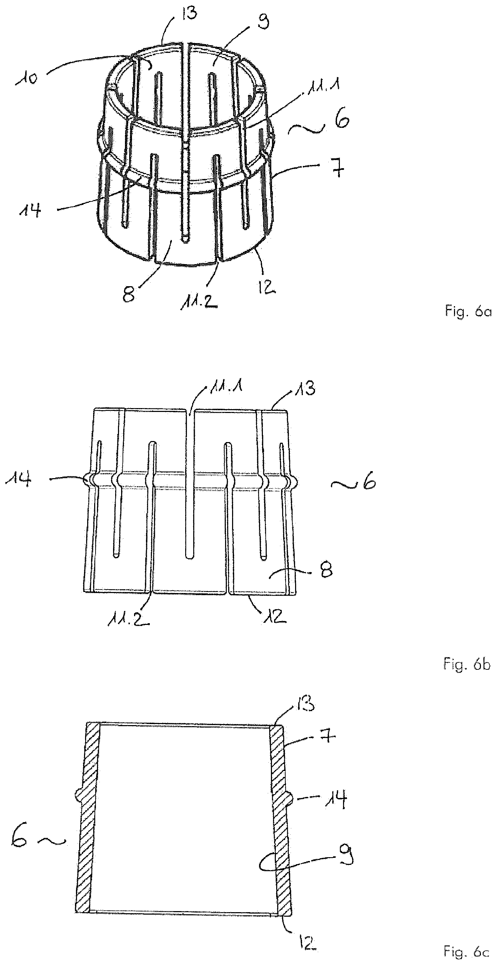

Die Fig. 6a -c zeigen eine konusförmige Spreizhülse 6, die mit einem Kupplungskörper 3 gemäß der in den Fig. 5a und b gezeigten Ausgestaltung kompatibel ist.

Die dargestellte Spreizhülse 6 hat einen Außenkonus 7 mit einer Mantelfläche 8 sowie einen Innenkonus 9 mit einer Wirkfläche 10, wobei die Konuswinkel des Außen- und Innenkonus gleich groß sind.

Auf der Mantelfläche 8 der Spreizhülse 6 ist eine radial umlaufende Wulst 14 eingerichtet, die zur Halterung der Spreizhülse 6 in der Fassung 4 des Kupplungskörpers 3 mit der in der Innenwandung 5 eingerichteten radial umlaufenden Nut 15 in Eingriff bringbar ist.

Um die Spreizhülse 6 in radialer Richtung aufweiten zu können, ist diese umlaufend in Längsrichtung eingeschlitzt, wobei die in Längsrichtung verlaufenden Schlitze 11.1 und 11.2 alternierend vom Rand 12 des breiteren Endes und vom Rand 13 des schmaleren Endes der Spreizhülse 6 ausgehen, so dass diese eine mäanderförmige Struktur aufweist, die dehnbar ist. Die Schlitze 11.1 und 11.2 durchteilen dabei die radial Wulst 14 in jeweilige Teilstücke.

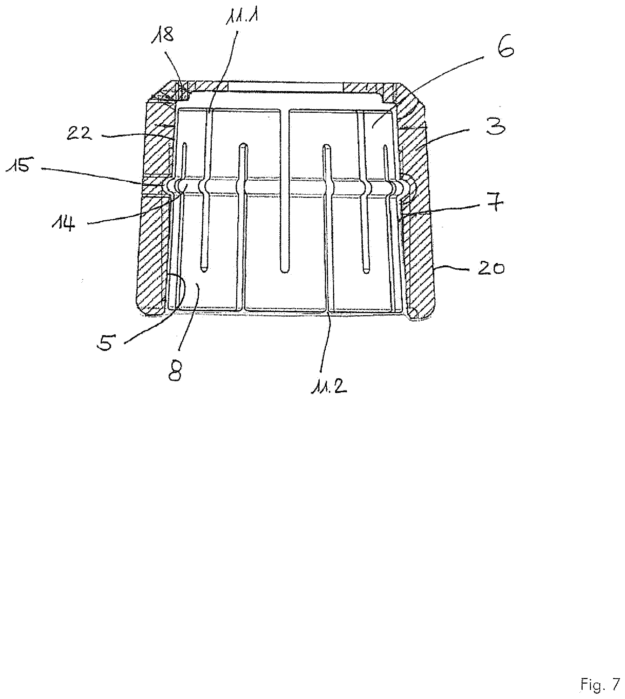

Die Fig. 7 zeigt die zweite Alternative einer Kupplungsvorrichtung, bei der die Spreizhülse 6 gemäß der Fig. 6a - c eingesetzt ist in die Fassung 4 eines Kupplungskörpers 3, wie er beispielhaft in den Fig. 5 a - b wiedergegeben ist. Die Spreizhülse 6 ist beweglich in der Fassung 4 gelagert und durch den Eingriff der Wulst 14 in die Nut 15 gegen ein Herausgleiten aus der Fassung 4 gesichert.

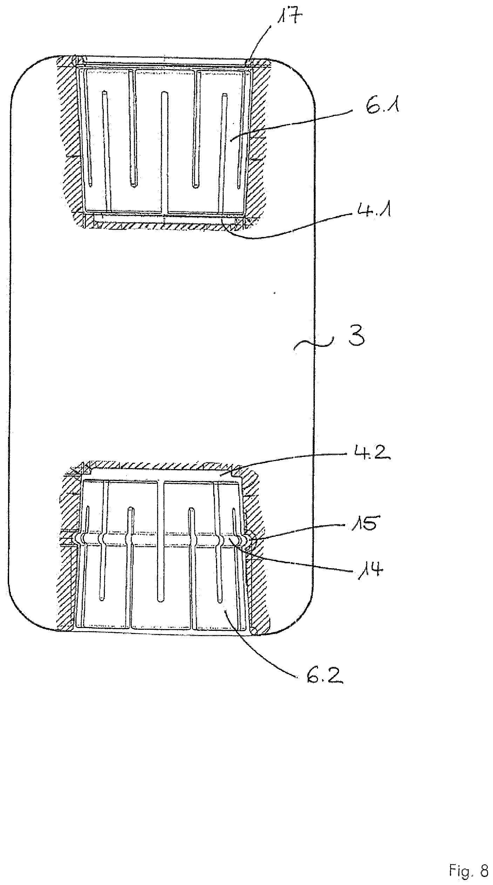

Die Fig. 8 zeigt eine Ausgestaltungsform einer erfindungsgemäßen Kupplungsvorrichtung, die zur Kopplung von zwei Prothesenkomponenten mit jeweils mindestens einem konusförmigen Endbereich eingesetzt werden kann.

Derartige Prothesenkomponenten sind insbesondere Knochennägel und Schäfte von Gelenkprothesen, so dass eine solche Kupplungsvorrichtung eingesetzt werden kann, um beispielsweise liegende Schäfte in dorsaler Richtung zu verlängern.

Zur Verlängerung des Schaftendes eines Hüftschaftes im Bereich des Femurs wird beispielsweise die Kupplungsvorrichtung mit der in der proximalen Fassung gelagerten Spreizhülse auf den Konus am Schaftende aufgesteckt. Zur Verbindung mit einem intramedullär gesetzten Knochennagel wird dann die in der dorsal eingerichteten Fassung gelagerte Spreizhülse auf den Konus des Knochennagels aufgeschoben, wodurch Hüftschaft und Knochennagel in longitudinaler Richtung beidseitig jeweils mittels eines selbsthemmenden Klemmsitzes miteinander gekoppelt werden.

Die in der Fig. 8 gezeigte Kupplungsvorrichtung umfasst hierzu einen zylinderförmigen Kupplungskörper 3, der in dieser Ausgestaltung geradlinig ausbildet ist und an dessen gegenüberliegenden Enden jeweils eine Fassung 4.1 bzw. 4.2 mit einer darin gelagerten Spreizhülse 6.1 bzw. 6.2 eingerichtet ist.

Die Kombination von Spreizhülse 6.1 und Fassung 4.1, die am oberen Ende des Kupplungskörpers 3 eingerichtet ist, entspricht einer Ausgestaltung wie sie zu Fig. 4 beschrieben wurde. Die am gegenüberliegenden Ende des Kupplungskörpers 3 befindliche Kombination von Spreizhülse 6.2 und Fassung 4.2 gibt eine Ausführungsvariante gemäß der Beschreibung zu Fig. 7 wieder.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

### Bezugszeichenliste:

- 1: erste Prothesenkomponente
- 2: Konus der ersten Prothesenkomponente
- 3: äußerer Kupplungskörper
- 4: Fassung
- 5: Innenwandung der Fassung
- 6: Spreizhülse
- 7: Außenkonus
- 8: Mantelfläche
- 9: Innenkonus
- 10: Wirkfläche
- 11: Schlitz
- 12: Rand am breiten Konusende der Spreizhülle
- 13: Rand am schmalen Konusende der Spreizhülle
- 14: Wulst auf der Mantelfläche der Spreizhülse
- 15: Nut in der Innenwandung der Fassung
- 16: Aufnahmeöffnung
- 17: radial umlaufende Hinterschneidung
- 18: stufenartige und radial umlaufende Einschnürung
- 19: zweite Prothesenkomponente
- 20: konusförmige Außenkontur der Kupplungsvorrichtung
- 21: konusförmigen Innenkontur in einer zweiten Prothesenkomponente
- 22: Mutterkonus (Innenkonus der Fassung)
- 23: zur Umgebung offene Bohrung im Kopfbereich der Fassung

## Patentansprüche

1. Kupplungsvorrichtung zur Verbindung von Prothesenkomponenten, mit welcher ein selbsthemmender Klemmsitz auf einem als Konus (2) ausgebildeten Ende einer in ein Knochengewebe eingesetzten ersten Prothesenkomponente (1) erzeugbar ist, wobei die Kupplungsvorrichtung einen äußeren Kupplungskörper (3) mit mindestens einer Fassung (4) mit einer konisch zulaufenden Innenwandung (5) und eine Spreizhülse (6) mit einer einen Außenkonus (7) bildenden Mantelfläche (8) und einer einen Innenkonus (9) bildenden Wirkfläche (10) umfasst, **dadurch gekennzeichnet, dass** die Spreizhülse (6) in der Fassung (4) um deren Längsachse rotierbar gelagert und zur Aufnahme des Konus (2) der ersten Prothesenkomponente (1) hergerichtet ist, und dass die Spreizhülse (6) zur Ausbildung des selbsthemmenden Klemmsitzes radial nach außen dehnbar ausgestaltet ist, so dass beim Einfügen des Konus (2) der ersten Prothesenkomponente (1) in die Spreizhülse (6) eine radiale Aufweitung der Spreizhülse (6) erfolgt, wodurch der selbsthemmende Klemmsitz zwischen dem Konus (2) der ersten Prothesenkomponente (1) und der Kupplungsvorrichtung herstellbar ist.

2. Kupplungsvorrichtung nach Anspruch 1, **dadurch gekennzeichnet, dass** die Spreizhülse (6) mindestens einen von einem Rand (12; 13) ausgehenden und in Längsrichtung der Spreizhülse (6) verlaufenden Schlitz (11) aufweist.

3. Kupplungsvorrichtung nach Anspruch 2, **dadurch gekennzeichnet, dass** die Spreizhülse (6) in Längsrichtung durchgehend geschlitzt ist.

4. Kupplungsvorrichtung nach einem der Ansprüche 1 oder 2, **dadurch gekennzeichnet, dass** umlaufend in der Spreizhülse (6) in Längsrichtung verlaufende Schlitze (6) eingearbeitet sind, die alternierend vom Rand (12) des breiteren Endes und vom Rand (13) des schmaleren Endes ausgehen, so dass die Spreizhülse (6) eine mäanderförmige Struktur aufweist.

5. Kupplungsvorrichtung nach einem der Ansprüche 1 bis 4, **dadurch gekennzeichnet, dass** die Konizität des Innenkonus (9) der Spreizhülse (6) ein geringfügig kleineres Maß als die Konizität des Konus (2) der ersten Prothesenkomponente (1) aufweist.

6. Kupplungsvorrichtung nach einem der Ansprüche 1 bis 5, **dadurch gekennzeichnet, dass** auf der Mantelfläche (8) der Spreizhülse (6) eine radial umlaufende Wulst (14) eingerichtet ist, die mit einer radial umlaufenden Nut (15) in der Innenwandung (5) der Fassung (4) in Eingriff bringbar ist.

7. Kupplungsvorrichtung nach einem der Ansprüche 1 bis 6, **dadurch gekennzeichnet, dass** in der Aufnahmeöffnung (6) der Fassung (4) eine radial umlaufende Hinterschneidung (17) eingerichtet ist.

8. Kupplungsvorrichtung nach einem der Ansprüche 1 bis 7, **dadurch gekennzeichnet, dass** im Bereich des schmalen Konusende der Fassung (4) auf der Innenwandung (5) eine stufenartige und radial umlaufende Einschnürung (18) eingerichtet ist.

9. Kupplungsvorrichtung nach einem der Ansprüche 1 bis 8, **dadurch gekennzeichnet, dass** der Kupplungskörper eine konusförmige Außenkontur (20) aufweist, die mit einer konusförmigen Innenkontur (21) in einer zweiten Prothesenkomponente (19) in einen selbsthemmenden Klemmsitz bringbar ist.

10. Kupplungsvorrichtung nach einem der Ansprüche 1 bis 8, **dadurch gekennzeichnet, dass** die Kupplungsvorrichtung einen zylinderförmigen Kupplungskörper (3) aufweist, an dessen Enden jeweils eine Fassung (4.1; 4.2) mit einer darin gelagerten Spreizhülse (6.1; 6.2) eingerichtet ist.

## Claims

1. A coupling device for connecting prosthesis components, with which a self-locking press fit can be created on an end, constructed as a cone (2), of a first prosthesis component (1) inserted into a bone tissue, the coupling device comprising an outer coupling body (3) having at least one socket (4) with a conically tapering inner wall (5) and an expansion sleeve (6) with a lateral surface (8) forming an outer cone (7) and an engaging surface (10) forming an inner cone (9), **characterized in that** the expansion sleeve (6) is mounted in the socket (4) such that it can rotate about its longitudinal axis and is arranged for accommodating the cone (2) of the first prosthesis component (1), and **in that** the expansion sleeve (6) is configured to be radially outwardly expandable for forming the self-locking press fit, so that a radial widening of the expansion sleeve (6) takes place during the insertion of the cone (2) of the first prosthesis component (1) into the expansion sleeve (6), thereby producing the self-locking press fit between the cone (2) of the first prosthesis component (1) and the coupling device.

2. The coupling device according to claim 1, **characterized in that** the expansion sleeve (6) has at least one slot (11) starting from one edge (12; 13) and running in the longitudinal direction of the expansion sleeve (6).

3. The coupling device according to claim 2, **characterized in that** the expansion sleeve (6) is slotted continuously in the longitudinal direction.

4. The coupling device according to one of claims 1 or 2, **characterized in that** slots (6) running in the longitudinal direction are incorporated circumferentially in the expansion sleeve (6), which slots start alternately from the edge (12) of the wider end and from the edge (13) of the narrower end, so that the expansion sleeve (6) has a meandering structure.

5. The coupling device according to one of claims 1 to 4, **characterized in that** the conicity of the inner cone (9) of the expansion sleeve (6) has a slightly smaller dimension than the conicity of the cone (2) of the first prosthesis component (1).

6. The coupling device according to one of claims 1 to 5, **characterized in that** a radially circumferential bead (14) is set up on the lateral surface (8) of the expansion sleeve (6), which bead can be brought into engagement with a radially circumferential groove (15) in the inner wall (5) of the socket (4).

7. The coupling device according to one of claims 1 to 6, **characterized in that** a radially circumferential undercut (17) is set up in the accommodating opening (6) of the socket (4).

8. The coupling device according to one of claims 1 to 7, **characterized in that** a stepped and radially circumferential constriction (18) is set up on the inner wall (5) in the region of the narrow cone end of the socket (4).

9. The coupling device according to one of claims 1 to 8, **characterized in that** the coupling body has a conical outer contour (20), which can be brought into a self-locking press fit with a conical inner contour (21) in a second prosthesis component (19).

10. The coupling device according to one of claims 1 to 8, **characterized in that** the coupling device has a cylindrical coupling body (3), at the ends of which a socket (4.1; 4.2) with an expansion sleeve (6.1; 6.2) mounted therein is set up in each case.

## Revendications

1. Dispositif d'accouplement, destiné à assembler des composants de prothèse, permettant de créer un ajustement serré autobloquant sur une extrémité conçue sous la forme d'un cône (2) d'un premier composant de prothèse (1) inséré dans un tissu osseux, le dispositif d'accouplement comprenant un organe de couplage (3) extérieur, pourvu d'au moins une monture (4) avec une paroi intérieure (5) pointue en forme de cône et une douille expansible (6) dotée d'une surface d'enveloppe (8) formant un cône extérieur (7) et d'une surface active (10) formant un cône intérieur (9), **caractérisé en ce que** la douille expansible (6) est logée dans la monture (4) en étant rotative autour de son axe longitudinal et est aménagée pour recevoir le cône (2) du premier composant de prothèse (1), et **en ce que** pour créer l'ajustement serré autobloquant, la douille expansible (6) est conçue en étant extensible en direction radiale vers l'extérieur, de sorte que lors de l'insertion du cône (2) du premier composant de prothèse (1) dans la douille expansible (6), il s'effectue un élargissement radial de la douille expansible (6), suite auquel l'ajustement serré autobloquant peut être créé entre le cône (2) du premier composant de prothèse (1) et le dispositif d'accouplement.

2. Dispositif d'accouplement selon la revendication 1, **caractérisé en ce que** la douille expansible (6) comporte au moins une fente (11) partant du bord (12 ; 13) et s'écoulant dans la direction longitudinale de la douille expansible (6).

3. Dispositif d'accouplement selon la revendication 2, **caractérisé en ce que** la douille expansible (6) est fendue en continu dans la direction longitudinale.

4. Dispositif d'accouplement selon l'une quelconque des revendications 1 ou 2, **caractérisé en ce qu'**en périphérie sur la douille expansible (6) sont incorporées des fentes (6) s'écoulant dans la direction longitudinale, qui partent alternativement du bord (12) de l'extrémité plus large et du bord (13) de l'extrémité plus étroite, de sorte que la douille expansible (6) présente une structure en forme de méandres.

5. Dispositif d'accouplement selon l'une quelconque des revendications 1 à 4, **caractérisé en ce que** la conicité du cône intérieur (9) de la douille expansible (6) est d'une dimension légèrement inférieure à celle de la conicité du cône (2) du premier composant de prothèse (1).

6. Dispositif d'accouplement selon l'une quelconque des revendications 1 à 5, **caractérisé en ce que** sur la surface d'enveloppe (8) de la douille expansible (6) est aménagé un bourrelet (14) périphérique qui est susceptible d'être amené en engagement avec une rainure (15) périphérique en direction radiale dans la paroi intérieure (5) de la monture (4).

7. Dispositif d'accouplement selon l'une quelconque des revendications 1 à 6, **caractérisé en ce que** dans l'orifice de logement (6) de la monture (4) est aménagée une contre-dépouille (17) périphérique en direction radiale.

8. Dispositif d'accouplement selon l'une quelconque des revendications 1 à 7, **caractérisé en ce que** dans la zone de l'extrémité étroite de la monture (4), sur la paroi intérieure (5) est aménagé un étranglement (18) échelonné et périphérique dans la direction radiale.

9. Dispositif d'accouplement selon l'une quelconque des revendications 1 à 8, **caractérisé en ce que** l'organe de couplage comporte un contour extérieur (20) de forme conique qui est susceptible d'être amené dans un ajustement serré autobloquant avec un contour intérieur (21) de forme conique dans un deuxième composant de prothèse (19).

10. Dispositif d'accouplement selon l'une quelconque des revendications 1 à 8, **caractérisé en ce que** le dispositif d'accouplement comporte un organe de couplage (3) de forme cylindrique, sur l'extrémité duquel est aménagée respectivement une monture (4.1 ; 4.2) pourvue d'une douille expansible (6.1 ; 6.2) logée dans celle-ci.
